# EUROPEAN PATENT APPLICATION

(11) **EP 1 882 672 A1**
(43) Date of publication of application: **30.01.2008**
(21) Application number: 06711727.5
(22) Date of filing: 16.01.2006
(51) Int. Cl.: C01B 4/00, B01J 23/44, C07B 59/00, C07C 51/36, C07C 57/30, C07C 209/36, C07C 211/48

(54) **METHOD FOR PRODUCING DEUTERIUM GAS AND CATALYTIC DEUTERATION METHOD USING DEUTERIUM GAS OBTAINED THEREBY**

(30) Priority: 28.01.2005 JP 2005021754
(71) Applicant: Wako Pure Chemical Industries, Ltd., Osaka-shi, Osaka 540-8605 (JP)
(72) Inventor: HIROTA, Kosaku, Gifu 5020003 (JP); SAJIKI, Hironao, Gifu 5020823 (JP); ITO, Nobuhiro, Saitama 3501101 (JP)
(74) Representative: Winter, Brandl, Fürniss, Hübner Röss, Kaiser, Polte Partnerschaft Patent- und Rechtsanwaltskanzlei
(86) International application number: PCT/JP2006/300446
(87) International publication number: WO 2006/080202

(57) **Abstract**

[PROBLEMS]

To provide a method for producing heavy hydrogen gas. which enables heavy hydrogen gas to be efficiently produced from a deuterated solvent as a reaction substrate.

[MEANS FOR SOLVING PROBLEMS]

(1) A method for producing heavy hydrogen gas, comprising bringing a deuterated solvent into contact with hydrogen gas under pressure in the coexistence with a catalyst selected from a palladium catalyst, a platinum catalyst, a nickel catalyst, a cobalt catalyst, an iridium catalyst, and a rhodium catalyst and a ruthenium catalyst which are not coordinated with a ligand, and (2) a catalytic deuteration method of a compound having a reducible functional group, comprising bringing the heavy hydrogen gas obtained by the above (1) into contact with the compound having a reducible functional group in the coexistence with a catalytic reduction catalyst.

## Description

### Technical Field

The present invention relates to a method for producing heavy hydrogen gas by bringing a deuterated solvent into contact with hydrogen gas under pressure in the coexistence with a catalyst selected from a palladium catalyst, a platinum catalyst, a nickel catalyst, a cobalt catalyst, an iridium catalyst, a rhodium catalyst not coordinated with a ligand and a ruthenium catalyst not coordinated with a ligand, and further a catalytic deuteration method of a compound having a reducible functional group using the heavy hydrogen gas obtained thereby.

### Background Art

A deuterated (deuterated and tritiated) compound is broadly used as a labeled compound and the like, in various fields such as life science, environmental science and material science. The deuterated compound, for example, is very useful in elucidation of a reaction mechanism, substance metabolism, etc. It is said that the compound is also useful as medicines, pesticides, organic EL materials, etc. due to change in stability and property of the compound itself by isotope effect thereof. It is also said that a tritiated compound is useful as a labeled compound in animal experiments to investigate absorption, distribution, concentration in blood, excretion, metabolism and the like of medicines. Therefore, researches using the deuterated (deuterated and tritiated) compound have been recently prevalent in these fields.

Various methods are available for producing the deuterated compound and among these, for example, a method of directly introducing into a final product by H-D exchange reaction, a catalytic deuteration method for a reducible functional group and the like are preferable. Heavy hydrogen gas (D₂, T₂) is used as a heavy hydrogen source in these reactions.

However, since D₂ gas, for example, is extremely expensive, deuterium oxide (D₂O), which is relatively inexpensive and easy to obtain, for example, has been tried to use as a heavy hydrogen source for producing heavy hydrogen gas.

As a method for producing heavy hydrogen gas using deuterium oxide, a method of electrolysis of deuterium oxide, for example, is known (Patent Literature 1). This method, however, has problems that a vast amount of energy is consumed, and use of deuterated alkali hydroxide (for example, KOD) is expensive and that it is necessary to separate heavy hydrogen gas from oxygen gas (O₂) produced as a by-product.

Another method for producing heavy hydrogen gas by reacting hydrogen gas (H₂) and deuterium oxide under pressure (2 MPa = about 20 atmosphere) using a ruthenium (II) complex and a rhodium (I) complex as a water-soluble complex catalyst has been proposed (Non-Patent Literature 1 and Non-Patent Literature 2). This method, however, has problems that it does not produce heavy hydrogen gas (D₂) but produces only DH gas, and that use of a complicated complex catalyst causes difficulties to operate and is expensive due to need of synthesis of the said catalyst in advance, and that it is difficult to recover and reuse a complex catalyst from deuterium oxide because the complex catalyst is soluble in water.

In addition, some of the present inventors have proposed a method for producing heavy hydrogen gas by reacting hydrogen gas and deuterium oxide under ordinary temperature and ordinary pressure in the presence of a palladium carbon (Pd/C) catalyst (Non-Patent Literature 3). The method, however, is hardly an efficient method due to a low ratio (production rate of heavy hydrogen) of the produced heavy hydrogen gas for deuterium oxide as a reaction substrate, although it can obtain pure heavy hydrogen gas.

In such a situation, it is desired to develop a method for efficiently producing heavy hydrogen gas from a deuterated solvent.

[Patent Literature 1]: U.S. Pat. No. 4,054,496
[Non-Patent Literature 1]: Gabor et al, Green Chem. 2003, Vol. 5, p. 213 - 217
[Non-Patent Literature 2]: Gabor et al, C. R. Acad. Sci. Paris, Ser. Iic: Chem. Vol. 3 (2003), p. 601 - 605
[Non-Patent Literature 3]: Organic Letters, 2004, Vol. 6, No. 20, p. 3521 - 3523

### Disclosure of the Invention

### Problem to be Solved by the Invention

The subject of the present invention is to provide a method which enables heavy hydrogen gas to be efficiently produced from a deuterated solvent as a reaction substrate.

### Means for Solving the Problem

The present invention relates to (1) a method for producing heavy hydrogen gas, comprising bringing a deuterated solvent into contact with hydrogen gas under pressure in the coexistence with a catalyst selected from a palladium catalyst, a platinum catalyst, a nickel catalyst, a cobalt catalyst, an iridium catalyst, a rhodium catalyst not coordinated with a ligand and a ruthenium catalyst not coordinated with a ligand, and (2) a catalytic deuteration method for a compound having a reducible functional group characterized by bringing the heavy hydrogen gas obtained in the above (1) into contact with the compound having a reducible functional group.

### Effect of the Invention

It is possible to obtain heavy hydrogen gas at a high production rate from a deuterated solvent using the method for producing heavy hydrogen gas of the present invention. When a compound having a reducible functional group (reaction substrate) is subjected to catalytic deuteration reaction in the presence of a catalytic reduction catalyst using the heavy hydrogen gas obtained in the method for producing heavy hydrogen gas of the present invention, as a result, it is possible to subject the reaction substrate efficiently to the catalytic deuteration reaction using a deuterated solvent such as deuterium oxide which is relatively inexpensive and available without purchasing expensive heavy hydrogen and also it is possible to produce the deuterated compound at a high deuteration ratio.

### Best Mode for Carrying out the Invention

In the present invention, heavy hydrogen means deuterium (D) or tritium (T) and deuteration means substitution with deuterium and tritium. Further, in the present specification, "production efficiency (or production rate) of heavy hydrogen (gas)" means the ratio of produced heavy hydrogen gas to a deuterated solvent. Furthermore, in the present specification, "deuteration ratio" means the ratio of the reaction site of a compound having a reducible functional group added with heavy hydrogen by catalytic reduction reaction using heavy hydrogen.

Heavy hydrogen gas in the method for producing heavy hydrogen gas of the present invention includes deuterium (D₂) or tritium (T₂).

In the case where the heavy hydrogen is deuterium, the deuterated solvent includes, for example, deuterium oxide (D₂O); organic solvents including deuterated alcohols such as deuterated methanol, deuterated ethanol, deuterated propanol, deuterated isopropanol, deuterated butanol, deuterated tert-butanol, deuterated pentanol, deuterated hexanol, deuterated heptanol, deuterated octanol, deuterated nonanol, deuterated decanol, deuterated undecanol and deuterated dodecanol; and deuterated carboxylic acids such as deuterated formic acid, deuterated acetic acid, deuterated propionic acid, deuterated butyric acid, deuterated isobutyric acid, deuterated valeric acid, deuterated isovaleric acid and deuterated pivalic acid. Among these, deuterium oxide is preferable in view of environmental aspect and workability. In the case where the heavy hydrogen is tritium, the deuterated solvent includes, for example, tritium oxide (T₂O) or the like. These deuterated solvents may be used alone or in combination of 2 or more solvents as appropriate.

A deuterated solvent may be one wherein at least one hydrogen atom in the molecule is deuterated. For example, deuterated alcohols wherein the hydrogen atom of the hydroxyl group is deuterated, or deuterated carboxylic acids wherein the hydrogen atom of the carboxyl group is deuterated, can be used.

A reaction solvent may be used if necessary in the method for producing heavy hydrogen of the present invention. The reaction solvent to be used if necessary may be any solvent unless it has an adverse effect on the production of heavy hydrogen gas in the present invention, and includes ethers such as dimethyl ether, diethyl ether, diisopropyl ether, ethyl methyl ether, tert-butyl methyl ether, 1,2-dimethoxyethane, oxirane, 1,4-dioxane, dihydropyran and tetrahydrofuran; and aliphatic hydrocarbons such as hexane, heptane, octane, nonane, decane and cyclohexane. These reaction solvents may be used alone or in combination of 2 or more solvents as appropriate.

The palladium catalyst to be used as a catalyst in the method for producing heavy hydrogen gas of the present invention includes one having 0 to 4, preferably 0 to 2 and more preferably 0 valence of a palladium atom.

The platinum catalyst includes one having usually 0 to 4, preferably 0 to 2 and more preferably 0 valence of a platinum atom.

The nickel catalyst includes one having usually 0 to 2 and preferably 0 valence of a nickel atom.

The cobalt catalyst includes one having usually 0 or 1 valence of a cobalt atom.

The iridium catalyst includes one having usually 0 to 4, preferably 0 to3 and more preferably 0 valence of an iridium atom.

The rhodium catalyst includes one having usually 0 or 4 and preferably 0 valence of a rhodium atom.

The ruthenium catalyst includes one having usually 0 to 4 and preferably 0 valence of a ruthenium atom.

In the case of palladium, platinum, nickel, cobalt, iridium, rhodium and ruthenium, the above catalysts may be a metal itself (simple metal), and a hydroxide, an oxide, a halide or an acetate of those metals. In the case of palladium, platinum, nickel, cobalt and iridium, the above catalysts may include in addition a compound coordinated with the following ligands. Among these, for example, a simple metal, metal catalysts, which are not coordinated with a ligand, such as a hydroxide, an oxide, a halide or an acetate of its metal are preferable. Further, these metals (simple metals), a metal hydroxide, a metal oxide, a metal halide, a metal acetate or a metal complex may be supported by various carriers.

The ligand of a metal catalyst which may be coordinated with a ligand includes, for example, 1,5-cyclooctadiene (COD), dibenzylideneacetone (DBA), bipyridine (BPY), phenanthroline (PHE), benzonitrile (PhCN), isocyanide (RNC), triethylarsine (As(Et)₃), acetylacetonate (acac), pentamethylcyclopentadienyl (Cp*) and ethylenediamine (EN); and an organic phosphine ligand such as dimethylphenylphosphine (P(CH₃)₂Ph), diphenylphosphinoferrocene (DPPF), trimethylphosphine (P(CH₃)₃), triethylphosphine (PEt₃), tri-tert-butylphosphine (P^{t}Bu₃), tricyclohexylphosphine (PCy₃), trimethoxyphosphine (P(OCH₃)₃), triethoxyphosphine (P(OEt)₃), tri-tert-butoxyphosphine (P(O^{t}Bu)₃), triphenylphosphine (PPh₃), 1,2-bis(diphenylphosphino)ethane (DPPE), triphenoxyphosphine (P(OPh)₃) and tri-o-tolylphosphine (P(o-tolyl)₃).

Specific examples of the palladium metal catalyst include, for example, Pd; palladium hydroxide catalysts such as Pd(OH)₂; palladium oxide catalysts such as PdO; halogenated palladium catalysts such as PdBr₂, PdCl₂ and Pdl₂; palladium acetate catalysts such as palladium acetate (Pd(OAc)₂) and palladium trifluoroacetate (Pd(OCOCF₃)₂); and palladium metal complex catalysts, which are coordinated with a ligand, such as Pd(RNC)₂Cl₂, Pd(acac)₂, diacetate-bis-(triphenylphosphine)palladium [Pd(OAc)₂(PPh₃)₂], Pd(PPh₃)₄, Pd₂(dba)₃, Pd(NH₃)₂Cl₂, Pd(CH₃CN)₂Cl₂, dichloro-bis-(benzonitrile)palladium [Pd(PhCN)₂Cl₂], Pd(dppe)Cl₂, Pd(dppf)Cl₂, Pd(PCy₃)₂Cl₂, Pd(PPhs)₂Cl₂, Pd[P(o-tolyl)₃]₂Cl₂, Pd(cod)₂Cl₂, Pd(PPh₃)(CH₃CN)₂Cl₂ and Pd(en).

Specific examples of the platinum metal catalyst include, for example, Pt; platinum oxide catalysts such as PtO₂, halogenated platinum catalysts such as PtCl₄, PtCb and K₂PtCl₄; and platinum metal complex catalysts, which are coordinated with a ligand, such as PtCl₂(cod), PtCl₂(dba), PtCl₂(PCy₃)₂, PtCl₂(P(OEt)₃)₂, PtCl₂(P(O^{t}Bu)₃)₂, PtCl₂(bpy), PtCl₂(phe), Pt(PPh₃)₄, Pt(cod)₂, Pt(dba)₂, Pt(bpy)₂ and Pt(phe)₂.

Specific examples of the nickel metal catalyst include, for example, Ni; nickel oxide catalysts such as NiO; halogenated nickel catalysts such as NiCl₂: and nickel metal complex catalysts, which are coordinated with a ligand, such as NiCl₂(dppe), NiCl₂(PPh₃)₂, Ni(PPh₃)₄, Ni(P(OPh)₃)₄ and Ni(cod)₂.

Specific examples of the cobalt metal catalyst include, for example, cobalt metal complex catalysts, which are coordinated with a ligand, such as Co(C₃H₅){P(OCH₃)₃}₃.

Specific examples of the iridium metal catalyst include, for example, Ir; iridium metal complex catalysts, which are coordinated with a ligand, such as Ir(cod)(acac) and Cp*(P(CH₃)₃)IrCl₂.

Specific examples of the rhodium metal catalyst include, for example, Rh.

Specific examples of the ruthenium metal catalyst include, for example, Ru.

The carrier, in the case where the above metal catalysts are supported on a carrier, includes, for example, carbon, alumina, silica gel, zeolite, molecular sieves, ion exchange resins and polymers. Among these, carbon and alumina are preferable and carbon is more preferable.

The ion exchange resin to be used as a carrier may be one having no adverse effect on deuteration of the present invention, and includes, for example, a cation exchange resin and an anion exchange resin.

The cation exchange resin includes, for example, a weakly acidic cation exchange resin and a strong acidic cation exchange resin. The anion exchange resin includes, for example, a weakly basic anion exchange resin and a strong basic anion exchange resin.

The ion exchange resin generally contains a polymer cross-linked with a bifunctional monomer as a skeleton polymer, to which an acidic group or a basic group is bonded. The acidic group and the basic group are exchanged by various cations and anions (counter ions), respectively.

Specific examples of the weakly acidic cation exchange resin include, for example, one obtained by hydrolysis of a polymer of an acrylic ester or a methacrylic ester, cross-linked by divinylbenzene.

Specific examples of the strong acidic cation exchange resin include, for example, one obtained by sulfonation of copolymer of a styrene-divinylbenzene.

Specific examples of the strong basic anion exchange resin include, for example, one obtained by bonding an amino group to an aromatic ring of copolymer of a styrene-divinylbenzene.

Strength of basicity of a basic anion exchange resin increases with an amino group bonded in the order of a primary amino group, a secondary amino group, a tertiary amino group and a quaternary ammonium salt.

The ion exchange resin available on the market may be used as well as the above ion exchange resins, as a carrier of a catalyst in the method for producing heavy hydrogen gas of the present invention.

The polymer used as a carrier is not especially limited unless it has an adverse effect on the method for producing heavy hydrogen gas of the present invention. Examples of such polymers include one obtained by polymerization or copolymerization of a monomer shown by the following general formula [1].

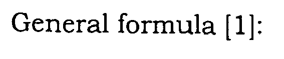

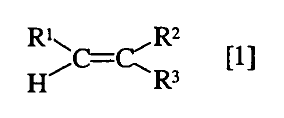

(wherein, R¹ represents a hydrogen atom, a lower alkyl group, a carboxyl group, a carboxyalkyl group, an alkoxycarbonyl group, a hydroxyalkoxycarbonyl group, a cyano group or a formyl group; R² represents a hydrogen atom, a lower alkyl group, a carboxyl group, an alkoxycarbonyl group, a hydroxyalkyloxycarbonyl group, a cyano group or a halogen atom; R³ represents a hydrogen atom, a lower alkyl group, a haloalkyl group, a hydroxyl group, an aryl group which may have a substituent, an aliphatic heterocyclic group, an aromatic heterocyclic group, a halogen atom, an alkoxycarbonyl group, a hydroxyalkyloxycarbonyl group, a sulfo group, a cyano group, a cyano-containing alkyl group, an acyloxy group, a carboxyl group, a carboxyalkyl group, an aldehyde group, an amino group, an aminoalkyl group, a carbamoyl group, an N-alkylcarbamoyl group or a hydroxyalkyl group; and R² and R³ may be bonded together with the adjacent -C=C - bond to form an aliphatic ring).

In the general formula [1], the lower alkyl group shown by R¹ to R³ may be straight chained, branched or cyclic, and includes, for example, an alkyl group having 1 to 6 carbon atoms, which is specifically exemplified by a methyl group, an ethyl group, a n-propyl group, an isopropyl group, a n-butyl group, an isobutyl group, a tert-butyl group, a sec-butyl group, a n-pentyl group, an isopentyl group, a sec-pentyl group, a tert-pentyl group, a neopentyl group, a 1-methylpentyl group, a n-hexyl group, an isohexyl group, a sec-hexyl group, a tert-hexyl group, a neohexyl group, a cyclopropyl group, a cyclobutyl group, a cyclopentyl group and a cyclohexyl group.

The carboxyalkyl group shown by R¹ and R² includes, for example, a group formed by replacing part of hydrogen atoms of the above lower alkyl group with a carboxyl group, which is specifically exemplified by, for example, a carboxymethyl group, a carboxyethyl group, a carboxypropyl group, a carboxybutyl group, a carboxypentyl group and a carboxyhexyl group.

The alkoxycarbonyl group shown by R¹ to R³ includes preferably, for example, one having 2 to 11 carbon atoms, which is specifically exemplified by, for example, a methoxycarbonyl group, an ethoxycarbonyl group, a propoxycarbonyl group, a butoxycarbonyl group, a pentyloxycarbonyl group, a hexyloxycarbonyl group, a cyclohexyloxycarbonyl group, a heptyloxycarbonyl group, an 2-ethylhexyloxycarbonyl group, an octyloxycarbonyl group, a nonyloxycarbonyl group and a decyloxycarbonyl group.

The hydroxyalkoxycarbonyl group shown by R¹ to R³ includes a group formed by replacing part of hydrogen atoms of the above alkoxycarbonyl group having 2 to 11 carbon atoms with a hydroxyl group, which is specifically exemplified by, for example, a hydroxymethoxycarbonyl group, a hydroxyethoxycarbonyl group, a hydroxypropoxycarbonyl group, a hydroxybutoxycarbonyl group, a hydroxypentyloxycarbonyl group, a hydroxyhexyloxycarbonyl group, a hydroxyheptyloxycarbonyl group, a hydroxyoctyloxycarbonyl group, a hydroxynonyloxycarbonyl group and a hydroxydecyloxycarbonyl group.

The halogen atom shown by R² and R³ includes, for example, fluorine, chlorine, bromine and iodine.

The haloalkyl group shown by R³ includes, for example, a group having 1 to 6 carbon atoms, which is formed by halogenating (for example, fluorinating, chlorinating, brominating and iodinating) the above lower alkyl group shown by R¹ to R³, which is specifically exemplified by, for example, a chloromethyl group, a bromomethyl group, a trifluoromethyl group, a 2-chloroethyl group, a 3-chloropropyl group, a 3-bromopropyl group, a 3,3,3-trifluoropropyl group, a 4-chlorobutyl group, a 5-chloropentyl group and a 6-chlorohexyl group.

The aryl group of the aryl group which may have a substituent includes, for example, a phenyl group, a tolyl group, a xylyl group and a naphthyl group, and said substituent includes, for example, an amino group, a hydroxyl group, a lower alkoxy group and a carboxyl group.

The lower alkoxy group as a substituent may be straight chained, branched or cyclic and includes usually a group having 1 to 6 carbon atoms, which is specifically exemplified by, for example, a methoxy group, an ethoxy group, a n-propoxy group, an isopropoxy group, a n-butoxy group, an isobutoxy group, a sec-butoxy group, a tert-butoxy group, a n-pentyloxy group, an isopentyloxy group, a sec-pentyloxy group, a tert-pentyloxy group, a neopentyloxy group, a n-hexyloxy group, an isohexyloxy group, a sec-hexyloxy group, a tert-hexyloxy group, a neohexyloxy group, a cyclopropoxy group, a cyclobutoxy group, a cyclopentyloxy group and a cyclohexyloxy group.

Specific examples of the substituted aryl group include, for example, an aminophenyl group, a toluidino group, a hydroxyphenyl group, a methoxyphenyl group, a tert-butoxyphenyl group and a carboxyphenyl group.

The aliphatic heterocyclic group includes, for example, a 5- or 6-membered ring having 1 to 3 hetero atoms such as a nitrogen atom, an oxygen atom and a sulfur atom, which is specifically exemplified by, for example, an 2-oxopyrrolidyl group, a piperidyl group, a piperidino group, a piperazinyl group and a morpholino group.

The aromatic heterocyclic group includes, for example, a 5- or 6-membered ring having 1 to 3 hetero atoms such as a nitrogen atom, an oxygen atom and a sulfur atom, which is specifically exemplified by, for example, a pyridyl group, an imidazolyl group, a thiazolyl group, a furyl group and a pyranyl group.

The cyano-containing alkyl group includes, for example, a group formed by replacing part of hydrogen atoms of the above lower alkyl group with cyano groups, which is specifically exemplified by, for example, a cyanomethyl group, a 2-cyanoethyl group, a 2-cyanopropyl group, a 3-cyanopropyl group, a 2-cyanobutyl group, a 4-cyanobutyl group, a 5-cyanopentyl group and a 6-cyanohexyl group.

The acyloxy group includes, for example, a group derived from a carboxylic acid having 2 to 20 carbon atoms, which is specifically exemplified by, for example, an acetyloxy group, a propionyloxy group, a butyryloxy group, a pentanoyloxy group, a nonanoyloxy group, a decanoyloxy group and a benzoyloxy group.

The aminoalkyl group includes a group formed by replacing part of hydrogen atoms of the above lower alkyl group with amino groups, and which is specifically exemplified by, for example, an aminomethyl group, an aminoethyl group, an aminopropyl group, an aminobutyl group, an aminopentyl group and an aminohexyl group.

The N-alkylcarbamoyl group includes a group formed by replacing part of hydrogen atoms of a carbamoyl group with alkyl groups, which is specifically exemplified by, for example, an N-methylcarbamoyl group, an N-ethylcarbamoyl group, an N-n-propylcarbamoyl group, an N-isopropylcarbamoyl group, an N-n-butylcarbamoyl group and an N-tert-butylcarbamoyl group.

The hydroxyalkyl group includes a group formed by replacing part of hydrogen atoms of the above lower alkyl group with hydroxyl groups, which is specifically exemplified by, for example, a hydroxymethyl group, a hydroxyethyl group, a hydroxypropyl group, a hydroxybutyl group, a hydroxypentyl group and a hydroxyhexyl group.

The aliphatic ring in the case where R¹ and R² are bonded together with the adjacent -C=C-bond to form an aliphatic ring, includes, for example, an unsaturated aliphatic ring having 5 to 10 carbon atoms,and may be monocyclic or polycyclic, which is specifically exemplified by, for example, a norbornene ring, a cyclopentene ring, a cyclohexene ring, a cyclooctene ring and a cyclodecene ring.

The specific examples of the monomer represented by the general formula [1] include ethylenically unsaturated aliphatic hydrocarbons having 2 to 20 carbon atoms such as ethylene, propylene, butylene and isobutylene; ethylenically unsaturated aromatic hydrocarbons having 8 to 20 carbon atoms such as styrene, 4-methylstyrene, 4-ethylstyrene and divinylbenzene; alkenyl esters having 3 to 20 carbon atoms such as vinyl formate, vinyl acetate, vinyl propionate and isopropenyl acetate; halogen-containing ethylenically unsaturated compounds having 2 to 20 carbon atoms such as vinyl chloride, vinylidene chloride, vinylidene fluoride and tetrafluoroethylene; ethylenically unsaturated carboxylic acids having 3 to 20 carbon atoms such as acrylic acid, methacrylic acid, itaconic acid, maleic acid, fumaric acid, crotonic acid, vinylacetic acid, allylacetic acid and vinylbenzoic acid (these acids may take a form of a salt of an alkali metal such as sodium and potassium or an ammonium salt); ethylenically unsaturated carboxylic esters such as methyl methacrylate, ethyl methacrylate, propyl methacrylate, butyl methacrylate, 2-ethylhexyl methacrylate, methyl acrylate, ethyl acrylate, propyl acrylate, butyl acrylate, 2-ethylhexyl acrylate, lauryl methacrylate, stearyl acrylate, methyl itaconate, ethyl itaconate, methyl maleate, ethyl maleate, methyl fumarate, ethyl fumarate, methyl crotonate, ethyl crotonate and methyl 3-butenoate; cyano-containing ethylenically unsaturated compounds having 3 to 20 carbon atoms such as acrylonitrile, methacrylonitrile and allyl cyanide; ethylenically unsaturated amide compounds having 3 to 20 carbon atoms such as acrylamide and methacrylamide; ethylenically unsaturated aldehydes having 3 to 20 carbon atoms such as acrolein and crotonaldehyde; ethylenically unsaturated sulfonic acids having 2 to 20 carbon atoms such as vinylsulfonic acid and 4-vinylbenzene sulfonic acid (these acids may take a form of a salt of an alkali metal such as sodium and potassium); ethylenically unsaturated aliphatic amines having 2 to 20 carbon atoms such as vinylamine and allylamine; ethylenically unsaturated aromatic amines having 8 to 20 carbon atoms such as vinylaniline; ethylenically unsaturated aliphatic heterocyclic amines having 5 to 20 carbon atoms such as N-vinylpyrrolidone and vinylpiperidine; ethylenically unsaturated alcohols having 3 to 20 carbon atoms such as allyl alcohol and crotyl alcohol; ethylenically unsaturated phenols having 8 to 20 carbon atoms such as 4-vinylphenol.

In a catalyst supported by a carrier, the content of palladium, platinum, nickel, cobalt, iridium, rhodium or ruthenium as a catalyst metal, is usually 1 to 99% by weight, preferably 1 to 50% by weight, more preferably 1 to 30% by weight, still more preferably 1 to 20% by weight, and particularly preferably 5 to 10% by weight based on the total weight of the catalyst.

In the method for producing heavy hydrogen gas of the present invention, among the above carrier-supported metal catalysts, a palladium carbon, a platinum carbon and a rhodium carbon are preferable, among these, a palladium carbon is particularly preferable.

The above catalysts may be used alone or in combination of 2 or more catalysts as appropriate in the method for producing heavy hydrogen gas of the present invention.

The amount of a catalyst to be used in the method for producing heavy hydrogen gas of the present invention is usually the so-called catalyst quantity, preferably in the order of 0.01 to 200% by weight, 0.01 to 100% by weight, 0.01 to 50% by weight, 0.01 to 20% by weight, 0.1 to 20% by weight, 1 to 20% by weight and 5 to 20% by weight, based on a deuterated solvent as a heavy hydrogen source, irrespective of whether the catalyst is supported by a carrier or not (when supported by a carrier, the amount of the catalyst is based on the weight of the catalyst supported by the carrier), and the upper limit content of the catalyst metal in said whole catalyst is preferably in the order of 20% by weight, 10% by weight, 5% by weight and 2% by weight, whereas the lower limit content is preferably in the order of, 0.0005% by weight, 0.005% by weight, 0.05% by weight and 0.5% by weight.

With regard to the reaction pressure in the method for producing heavy hydrogen gas of the present invention, the lower limit is usually 2 atmosphere, preferably in the order of 3 atmosphere, 4 atmosphere and 5 atmosphere, whereas the upper limit is usually 100 atmosphere, preferably in the order of 50 atmosphere, 30 atmosphere, 20 atmosphere and 15 atmosphere.

The more deuterated solvent is used in the method for producing heavy hydrogen gas of the present invention, the more heavy hydrogen gas can be produced. From the economical stand point, however, the lower limit of the amount of the deuterated solvent to be used is usually 0.1 molar times and preferably in the order of 0.5 molar times and 1 molar time, and the upper limit is usually 50 molar times and preferably in the order of 40 molar times, 30 molar times and 20 molar times, relative to the hydrogen gas to be used.

In the method for producing heavy hydrogen gas of the present invention, the reaction is carried out under a sealed condition and the reaction system may be pressurized by adding hydrogen gas.

The reaction system may be further added with an inert gas such as nitrogen gas and argon gas.

When the method for producing heavy hydrogen gas of the present invention is carried out under a sealed condition, a vessel such as a pressure vessel usually used for pressurized reaction may be used. The vessel may be selected as appropriate from commercially available pressure vessels having the volume that meets the above reaction pressure and the amount of hydrogen gas to be used.

With regard to the reaction temperature in the method for producing heavy hydrogen gas of the present invention, the lower limit is usually 0°C, preferably in the order of 5°C, 10°C, 15°C and 20°C, whereas the upper limit is usually 100°C, preferably in the order of 70°C and 50°C.

With regard to the reaction time in the method for producing heavy hydrogen gas of the present invention, the lower limit is usually 6 hours, preferably in the order of 12 hours, 24 hours, 48 hours and 72 hours, whereas the upper limit is usually 120 hours, preferably in the order of 108 hours and 96 hours.

The method for producing heavy hydrogen gas of the present invention will be described as follows, taking as an example the case where a palladium catalyst as the catalyst, deuterium oxide as the deuterated solvent and a 96-mL autoclave as the pressure vessel are used.

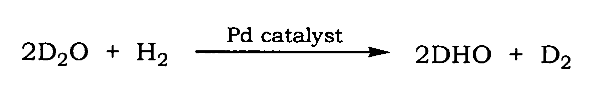

For example, the palladium catalyst (the palladium metal is 1 to 2% by weight based on the deuterium oxide) is added to 1 to 2 mL of the deuterium oxide (about 1 to 6 molar times relative to the hydrogen gas). The reaction system is sealed and pressurized (2 to 11 atmosphere) with hydrogen gas. The reaction is carried out under stirring at about 20 to 50°C for about 24 to 96 hours to obtain heavy hydrogen gas.

The obtained amount of heavy hydrogen gas is determined by measuring the amount of DHO produced at the same time using ¹H-NMR.

The ratio of D₂ produced from D₂O as the reaction substrate is referred to as "production efficiency (or production rate) of D₂" and calculated as follows.

Production rate of D₂ = DHO/ (DHO + D₂O) % by mole

The obtained heavy hydrogen gas may be collected in, for example, a balloon and used for various reactions such as catalytic deuteration reaction.

The method for producing heavy hydrogen gas of the present invention enables D₂ to be efficiently produced from relatively inexpensive D₂O without causing the problems existing in conventional methods, for example, that electrolysis methods consume much energy leading to high cost, and that use of complicated complex agents which are necessary to be synthesized in advance makes the process complicated and costly.

Further, the method for producing heavy hydrogen gas of the present invention also enables heavy hydrogen gas to be produced in a high production efficiency from a deuterated solvent as a raw material.

Therefore, for example, when an desired substrate is subjected to catalytic deuteration reaction using the heavy hydrogen gas obtained by the method for producing heavy hydrogen gas of the present invention, the reaction site of the substrate can be catalytically reduced to form the deuterated compound efficiently.

The catalytic deuteration method of the present invention may bring a compound having a reducible functional group into contact with the heavy hydrogen gas obtained by the method for producing heavy hydrogen gas of the present invention, in a reaction solvent if necessary, for example, in the coexistence with a catalytic reduction catalyst.

The reducible functional group of a compound having a reducible functional group in the catalytic deuteration method of the present invention may be any group as long as it is a group that can be reduced (hydrogenated) by an ordinary catalytic reduction. Said reducible functional group includes, for example, a carbon-carbon double bond, a carbon-carbon triple bond, an epoxy group, a halogen atom, a nitrile group, a hydroxyl group bonding to a carbon atom directly bonding to an aromatic ring (hydroxyl group bonding to benzyl position), a carbonyl group, an imino group, an aromatic ring and a cyclic alkyl group having 3 to 4 carbon atoms.

The carbon-carbon double bond and the carbon-carbon triple bond represent a reactive double bond and a reactive triple bond, which can be reduced in an ordinary catalytic reduction, respectively.

The halogen atom as a reducible functional group includes, for example, a fluorine atom, a chlorine atom, a bromine atom and an iodine atom.

The aromatic ring and the aromatic ring of a hydroxyl group bonding to a carbon atom directly bonding to an aromatic ring may be a monocyclic ring or a condensed polycyclic ring. The condensed polycyclic ring may be derived from the condensation of two aromatic rings or the condensation of an aromatic ring and an aliphatic ring, and may have a plane structure or a steric structure.

Specific examples of the above aromatic rings include, for example, benzene, naphthalene, anthracene and phenanthrene.

The cyclic alkyl group includes a group having 3 to 4 carbon atoms, which is specifically exemplified by, for example, a cyclopropyl group and a cyclobutyl group.

The compound having a reducible functional group in the catalytic deuteration method of the present invention may be any compound as long as it has at least one of the above reducible functional groups therein.

The catalyst for catalytic reduction includes all catalysts which are usually used in catalytic reduction, such as a palladium catalyst, a platinum catalyst, a nickel catalyst, a rhodium catalyst, a ruthenium catalyst and a rhenium catalyst.

The palladium catalyst includes one having 0 to 4, preferably 0 to 2 and more preferably 0 valence of a palladium atom.

The platinum catalyst includes one having usually 0 to 4, preferably 0 to 2 and more preferably 0 valence of a platinum atom.

The nickel catalyst includes one having usually 0 to 2 and preferably 0 valence of a nickel atom.

The rhodium catalyst includes one having usually 0 or 4 and preferably 0 valence of a rhodium atom.

The ruthenium catalyst includes one having usually 0 to 4 and preferably 0 valence of a ruthenium atom.

The rhenium catalyst includes one having usually 0 to 7, preferably 0 to 3 and more preferably 0 valence of a rhenium atom.

The above catalysts may be a metal itself of palladium, platinum, nickel, rhodium, ruthenium or rhenium, and an oxide, a halide and an acetate, or a catalyst coordinated with a ligand, of these metals. These catalysts such as a metal, a metal oxide, a halide, an acetate, a Raney catalyst or a metal complex may be supported by various carriers.

The ligand of a metal catalyst that may be coordinated with a ligand includes a ligand similar to the specific examples of the ligand, which may be coordinated to a catalyst in the method for producing heavy hydrogen gas of the present invention.

Specific examples of the palladium metal catalyst and the platinum metal catalyst include a metal catalyst similar to the specific examples of the palladium metal catalyst and the platinum metal catalyst respectively in the method for producing heavy hydrogen gas of the present invention.

Specific examples of the nickel metal catalyst include, for example, the specific examples of the nickel metal catalyst in the method for producing heavy hydrogen gas of the present invention and Raney nickel.

Specific examples of the rhodium metal catalyst include, for example, Rh and a rhodium metal complex catalyst coordinated with a ligand such as RhCl(PPh₃)₃.

Specific examples of the ruthenium metal catalyst include, for example, Ru and a ruthenium metal complex catalyst coordinated with a ligand such as RuCl₂(PPh₃)₃.

Specific examples of the rhenium metal catalyst include, for example, Re, a rhenium oxide catalyst such as ReO₃ and a rhenium metal complex catalyst coordinated with a ligand such as Re(CH₃)Cp*(NO)PPh₃.

The carrier, in the case where the above metal catalysts are supported on a carrier, includes, for example, carbon, alumina, silica gel, zeolite, molecular sieves, ion exchange resins and polymers.

Specific examples of the ion exchange resins and the polymers, to be used as a carrier include similar one to the specific examples of the ion exchange resins and the polymers, exemplified as a carrier in the method for producing heavy hydrogen gas of the present invention.

In a catalytic reduction catalyst supported by a carrier, the content of palladium, platinum, nickel, rhodium, ruthenium or rhenium as a catalyst metal is usually 1 to 99% by weight, preferably 1 to 50% by weight, more preferably 1 to 30% by weight, still more preferably 1 to 20% by weight, and particularly preferably 5 to 10% by weight, based on the total weight of the catalyst.

The amount of a catalytic reduction catalyst to be used in the catalytic deuteration method of the present invention is usually the so-called catalyst quantity, preferably in the order of 0.01 to 200% by weight, 0.01 to 100% by weight, 0.01 to 50% by weight, 0.01 to 20% by weight, 0.1 to 20% by weight, 1 to 20% by weight and 10 to 20% by weight based on a compound having a reducible functional group as a reaction substrate, irrespective of whether the catalyst is supported by a carrier or not (when supported by a carrier, the amount of the catalyst is based on the weight of the catalyst supported by the carrier), and the upper limit content of the catalyst metal in said whole catalyst is preferably in the order of 20% by weight, 10% by weight, 5% by weight and 2% by weight, whereas the lower limit content is preferably in the order of 0.0005% by weight, 0.005% by weight, 0.05% by weight and 0.5% by weight.

With regard to the amount of use of the heavy hydrogen gas obtained in the method for producing heavy hydrogen gas of the present invention in the catalytic deuteration method of the present invention, the lower limit of the amount of use is usually 1 molar time and preferably in the order of 2 molar times, 3 molar times and 4 molar times, and the upper limit is usually 100 molar times and preferably in the order of 50 molar times, 30 molar times and 10 molar times, as large as the theoretical amount of a reducible functional group in the reaction substrate.

The reaction solvents may be used in the catalytic deuteration method of the present invention. Such reaction solvents include any solvent unless it has an adverse effect on the catalytic deuteration method of the present invention, in other words, for example, unless it is catalytically reduced, and which is specifically exemplified by organic solvents including aliphatic hydrocarbons having 5 to 11 carbon atoms such as pentane, hexane, heptane, octane, nonane, decane, undecane, dodecane, tridecane, tetradecane, cyclopentane, cyclohexane and cycloheptane; aromatic hydrocarbons such as benzene and naphthalene; alkyl-substituted aromatic hydrocarbons such as toluene, xylene, mesitylene, ethylbenzene, propylbenzene, cumene, butylbenzene, isobutylbenzene, tert-butylbenzene, pentylbenzene and hexylbenzene; alkoxy-substituted aromatic hydrocarbons such as anisole, ethoxybenzene, propoxybenzene; alcohols such as methanol, ethanol, propanol, butanol, pentanol, hexanol, heptanol, octanol, nonanol, decanol and benzyl alcohol; aliphatic carboxylic esters such as methyl formate, ethyl formate, methyl acetate, ethyl acetate, butyl acetate, methyl propionate, ethyl propionate, butyl propionate and ethyl butyrate; aromatic carboxylic esters such as methyl benzoate, ethyl benzoate, propyl benzoate and butyl benzoate; and ethers such as dimethyl ether, methyl ethyl ether, diethyl ether, diisopropyl ether, tert-butyl methyl ether, tetrahydrofuran, tetrahydropyran, 1,4-dioxane and cyclopentyl phenyl ether; and water. As described above, the reaction solvent includes solvents similar to the specific examples of the deuterated solvents in the method for producing heavy hydrogen gas of the present invention. These solvents are selected as appropriate according to the kind of reaction substrates, the reaction temperature or the objective reaction time and may be used alone or in combination of 2 or more solvents as appropriate.

In the catalytic deuteration of the present invention, a compound having a reducible functional group as a reaction substrate may not be entirely dissolved and may be suspended in the above reaction solvent.

With regard to the reaction temperature in the catalytic deuteration method of the present invention, the lower limit is usually 0°C, preferably in the order of 5°C, 10°C, 15°C and 20°C, whereas the upper limit is usually 80°C, preferably in the order of 60°C and 40°C.

With regard to the reaction time in the catalytic deuteration method of the present invention, the lower limit is usually 10 minutes, preferably in the order of 1 hour, 2 hours and 6 hours, whereas the upper limit is usually 72 hours, preferably in the order of 48 hours and 24 hours.

The reaction pressure in the catalytic deuteration method of the present invention is usually ordinary pressure to 10 atmosphere, preferably ordinary pressure to 5 atmosphere and more preferably ordinary pressure to 3 atmosphere.

The catalytic deuteration method of the present invention will be described as follows, taking as an example the case where a palladium catalyst as the catalyst is used. The heavy hydrogen gas to be used are obtained by using a palladium catalyst as the catalyst and deuterium oxide as the deuterated solvent in the method for producing heavy hydrogen gas of the present invention.

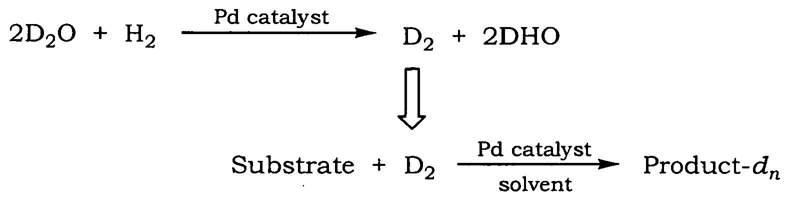

For example, the above reaction substrate (Substrate) and a palladium catalyst (the content of palladium metal is 0.05 to 3% by weight based on the substrate) are added to a reaction solvent. The system is replaced with the heavy hydrogen gas obtained in the method for producing heavy hydrogen gas of the present invention (for example, a palladium catalyst as the catalyst and deuterium oxide as the deuterated solvent are used) and then subjected to reaction under stirring at about 10 to 40°C for about 1 to 10 hours. After termination of the reaction, the reaction solution is filtered to remove the catalyst, purified and then subjected to structural analysis by ¹H-NMR.

According to the catalytic deuteration method of the present invention, D₂ gas, which is expensive and difficult to obtain, can be easily produced from D₂O, which is relatively inexpensive and easy to obtain, and then a reaction substrate can be catalytically deuterated efficiently by using the produced D₂ gas.

Instead of hydrogen gas to be used in an ordinary method (for example, Organic Letters, Vol.6, No.26, 2004 etc.) for producing a secondary amine compound by reducible monoalkylation reaction, use of the heavy hydrogen gas obtained by the method for producing heavy hydrogen gas of the present invention can produce easily a deuterated secondary amine compound.

More specifically, a nitro compound or an amine compound may be subjected to reaction with a nitrile compound, in the presence of a catalyst such as, for example, a palladium metal catalyst and a rhodium metal catalyst, in the coexistence of the heavy hydrogen gas obtained by the method for producing heavy hydrogen gas of the present invention.

The above reaction is shown below in detail.

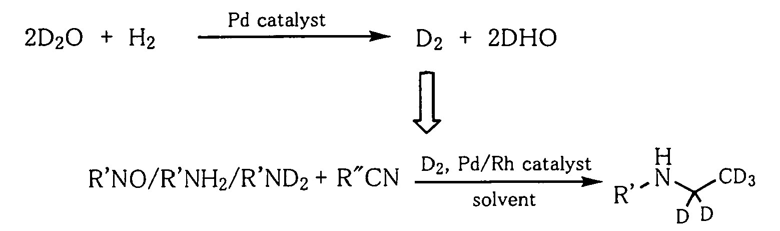

(wherein, R' represents an alkyl group or an aryl group, which may be deuterated; R" represents an alkyl group which may be deuterated)

More specifically, for example, a nitro compound (R'NO) or an amine compound (R'NH₂ or R'ND₂), which may be deuterated, is added to a nitrile compound (R"CN) which may be deuterated, in a reaction solvent if necessary, in the presence of a palladium catalyst or a rhodium catalyst (the content of palladium metal or rhodium metal is 0.05 to 3% by weight based on the substrate). The system is replaced with the heavy hydrogen gas obtained by the method for producing heavy hydrogen gas of the present invention (for example, in the case where palladium catalyst as the catalyst and deuterium oxide as the deuterated solvent are used) and then the compounds are subjected to reaction under intense stirring at about 10 to 40°C for about 1 to 24 hours. After termination of the reaction, the reaction solution is filtered to remove the catalyst, purified and then subjected to structural analysis by ¹H-NMR.

The nitro compound which may be deuterated includes, for example, compounds shown by R'NO.

The amine compound which may deuterated includes, for example, compounds shown by R'NH₂ and R'ND₂.

The nitrile compound which may be deuterated includes, for example, compounds shown by R"CN.

The alkyl groups of the alkyl group which may be deuterated, shown by R' and R" may be straight chained, branched or cyclic, and include usually groups having 1 to 15 carbon atoms, which is specifically exemplified by, for example, a methyl group, an ethyl group, a n-propyl group, an isopropyl group, a n-butyl group, an isobutyl group, a sec-butyl group, a tert-butyl group, a n-pentyl group, an isopentyl group, a sec-pentyl group, a tert-pentyl group, a neopentyl group, a n-hexyl group, an isohexyl group, a sec-hexyl group, a tert-hexyl group, a neohexyl group, a n-heptyl group, an isoheptyl group, a sec-heptyl group, a tert-heptyl group, a neoheptyl group, a n-octyl group, an isooctyl group, a sec-octyl group, a tert-octyl group, a neooctyl group, a n-nonyl group, an isononyl group, a sec-nonyl group, a tert-nonyl group, a neononyl group, a n-decyl group, an isodecyl group, a sec-decyl group, a tert-decyl group, a neodecyl group, an undecyl group, an isoundecyl group, a sec-undecyl group, a tert-undecyl group, a neoundecyl group, a n-dodecyl group, an isododecyl group, a sec-dodecyl group, a tert-dodecyl group, a neododecyl group, a n-tridecyl group, an isotridecyl group, a sec-tridecyl group, a tert-tridecyl group, a neotridecyl group, a n-tetradecyl group, an isotetradecyl group, a sec-tetradecyl group, a tert-tetradecyl group, a neotetradecyl group, a n-pentadecyl group, an isopentadecyl group, a sec-pentadecyl group, a tert-pentadecyl group, a neopentadecyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group and a cyclododecyl group.

The aryl group of the aryl group which may be deuterated, shown by R' includes, for example, groups having 6 to 14 carbon atoms, which is specifically exemplified by, for example, a phenyl group, a naphthyl group, a phenanthryl group and an anthryl group.

The alkyl group which was deuterated, shown by R' and R" includes groups formed by replacing a part or all of hydrogen atoms of the above alkyl group of the alkyl group which may be deuterated, shown by R' and R", with heavy hydrogen atoms.

The aryl group of the aryl group which was deuterated, shown by R' includes groups formed by replacing a part or all of hydrogen atoms of the above aryl group of the aryl group which may be deuterated, shown by R', with heavy hydrogen atoms.

The palladium metal catalyst includes one having 0 to 4, preferably 0 to 2 and more preferably 0 valence of a palladium atom.

The rhodium metal catalyst includes one having usually 0 or 4, preferably 0 valence of a rhodium atom.

The catalyst may be selected as appropriate according to reaction substrates and reaction conditions. It is preferable to select a palladium metal catalyst when a nitro compound which may be deuterated or an aromatic amino compound which may be deuterated, wherein R' is an aryl group which may be deuterated, is used as the reaction substrate, whereas it is preferable to select a rhodium metal catalyst when an aliphatic amino compound which may be deuterated, wherein R' is an alkyl group whicn may be deuterated, is used as the reaction substrate.

The above catalysts may be a metal itself, a metal oxide, a metal halide and a metal acetate, and one coordinated with a ligand. Further, a metal itself, a metal oxide, a halide, an acetate, a Raney catalyst or a metal complex may be supported by various carriers, also.

The ligand of a palladium catalyst or a rhodium catalyst, which may be coordinated with a ligand includes a ligand similar to the specific examples of the ligand which may be coordinated to a catalyst in the method for producing heavy hydrogen gas of the present invention.

Specific examples of the palladium metal catalyst include a catalyst similar to the specific examples of the palladium metal catalyst in the method for producing heavy hydrogen gas of the present invention.

Specific examples of the rhodium metal catalyst include a catalyst similar to the specific examples of the rhodium metal catalyst in the method for producing heavy hydrogen gas of the present invention.

The carrier, in the case where the above metal catalysts are supported on a carrier, includes, for example, carbon, alumina, silica gel, zeolite, molecular sieves, ion exchange resins and polymers.

Specific examples of the ion exchange resins and the polymers to be used as a carrier include similar one to the specific examples of the ion exchange resins and the polymers exemplified as a carrier in the method for producing heavy hydrogen gas of the present invention.

In a catalytic reduction catalyst supported by a carrier, the rate of palladium or rhodium, as a catalyst metal is usually 1 to 99% by weight, preferably 1 to 50% by weight, more preferably 1 to 30% by weight, still more preferably 1 to 20% by weight, and particularly preferably 5 to 10% by weight, based on the total weight of the catalyst.

The amount of a catalyst to be used in said method for producing a secondary amine compound is usually the so-called catalyst quantity, preferably in the order of 0.01 to 200% by weight, 0.01 to 100% by weight, 0.01 to 50% by weight, 0.01 to 20% by weight, 0.1 to 20% by weight, 1 to 20% by weight and 10 to 20% by weight, based on a nitro compound or amine compound, which may be deuterated as a reaction substrate, irrespective of whether the catalyst is supported by a carrier or not (when supported by a carrier, the amount of the catalyst is based on the weight of the catalyst supported by the carrier), and the upper limit content of the catalyst metal in said whole catalyst is preferably in the order of 20% by weight, 10% by weight, 5% by weight and 2% by weight, whereas the lower limit content is preferably in the order of 0.0005% by weight, 0.005% by weight, 0.05% by weight and 0.5% by weight.

In the method for producing a secondary amine compound related to the present invention, with regard to the amount of use of the heavy hydrogen gas obtained by the method for producing heavy hydrogen gas of the present invention, the lower limit of the amount of use is usually 1 molar time and preferably in the order of 2 molar times, 3 molar times and 4 molar times, and the upper limit is usually 100 molar times and preferably in the order of 50 molar times, 30 molar times and 10 molar times, relative to the theoretical amount of a nitro compound or amino compound, which may be deuterated in the reaction substrate.

The reaction solvent for use in the method for producing a secondary amine compound related to the present invention is not necessary to be used when a nitrile compound, an amino compound or a nitrile compound, which may be deuterated, as the reaction substrate, is liquid, but preferably is used when these compounds are solid. The above reaction solvent includes any solvent unless it has an adverse effect on said reaction for producing a secondary amine compound, which is specifically exemplified by, for example, organic solvents including aliphatic hydrocarbons having 5 to 11 carbon atoms such as pentane, hexane, heptane, octane, nonane, decane, undecane, dodecane, tridecane, tetradecane, cyclopentane, cyclohexane and cycloheptane; aromatic hydrocarbons such as benzene and naphthalene; alkyl-substituted aromatic hydrocarbons such as toluene, xylene, mesitylene, ethylbenzene, propylbenzene, cumene, butylbenzene, isobutylbenzene, tert-butylbenzene, pentylbenzene and hexylbenzene; alkoxy-substituted aromatic hydrocarbons such as anisole, ethoxybenzene and propoxybenzene; alcohols such as methanol, ethanol, propanol, butanol, pentanol, hexanol, heptanol, octanol, nonanol, decanol and benzyl alcohol; aliphatic carboxylic esters such as methyl formate, ethyl formate, methyl acetate, ethyl acetate, butyl acetate, methyl propionate, ethyl propionate, butyl propionate and ethyl butyrate; aromatic carboxylic esters such as methyl benzoate, ethyl benzoate, propyl benzoate and butyl benzoate; and ethers such as dimethyl ether, methyl ethyl ether, diethyl ether, diisopropyl ether, tert-butyl methyl ether, tetrahydrofuran, tetrahydropyran, 1,4-dioxane and cyclopentyl phenyl ether; and water. As described above, the reaction solvent includes solvents similar to the specific examples of the deuterated solvents in the method for producing heavy hydrogen gas of the present invention. These solvents are selected as appropriate according to the kind of reaction substrates, the reaction temperature or the objective reaction time and may be used alone or in combination of 2 or more solvents as appropriate.

Thus, the reducible monoalkylation reaction using the heavy hydrogen gas obtained by the method for producing heavy hydrogen gas of the present invention can produce easily D₂ gas from D₂O, which is relatively inexpensive and easy to obtain, without using D₂ gas, which is expensive and difficult to obtain, and then can produce efficiently a deuterated secondary amine compound by using the obtained D₂ gas.

The present invention will be described with the following example and comparative examples in more detail, but is not limited thereto at all.

### Examples

### Example 1. Synthesis of heavy hydrogen gas of the present invention

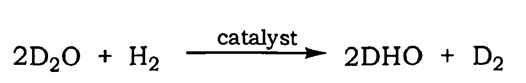

In a 96-mL autoclave serving as a pressure vessel, 1 mL (55 mmol) of deuterium oxide (D₂O) and 10 mg of 10% palladium carbon (Pd/C) were added, and the reaction system was pressurized with hydrogen (hydrogen pressure: 5 atmosphere, system pressure: 6 atmosphere in conjunction with atmosphere pressure) (H₂: 25.4 mmol) and subjected to reaction under stirring at room temperature for about 48 hours. After termination of the reaction, the amount of produced DHO was measured by ¹H-NMR, and the amount of D₂ produced from DHO and the D₂ production rate (DHO/D₂O + DHO) were calculated. These results are shown in Table 1.

### Examples 2 and 3. Synthesis of heavy hydrogen gas of the present invention

Heavy hydrogen gas was produced similarly as in Example 1 except that the amount of deuterium oxide, the amount of hydrogen gas, the amount of the catalyst, the reaction pressure and the reaction time were set as shown in Table 1. The results are also shown in Table 1.

### Comparative Example 1.

Heavy hydrogen gas was produced similarly as in Example 1 except that it was produced under atmospheric pressure (1 atmosphere, H₂: 89 mmol). The results are also shown in Table 1.

### Comparative Example 2.

Heavy hydrogen gas was produced similarly as in Example 1 except that any catalyst was not used. The results are also shown in Table 1.

**[Table 1]**

| | Reaction Conditions | | | | | Reaction Products | | D₂ Production rate (mol%) |
|---|---|---|---|---|---|---|---|---|
| | 10%Pd/C (mg) | Pres. (atm) | Time | H₂ (mmol) | D₂O (mmol) | DHO (mmol) | D₂ (mmol) | |
| Exam. 1 | 10 | 6 | 48h | 25.4 | 55 | 31.5 | 15.6 | 57 |
| Exam. 2 | 20 | 6 | 24h | 25.4 | 55 | 36.6 | 18.1 | 66.1 |
| Exam. 3 | 10 | 11 | 24h | 46.7 | 55 | 52 | 25.8 | 94.1 |
| Comp. Exam.1 | 10 | 1 | 72h | 89 | 55 | 7.41 | 3.57 | 13.4 |
| Comp. Exam.2 | none | 6 | 48h | 25.4 | 55 | 0.8 | 0.2 | 1.4 |

| | | | | | | | | |
|---|---|---|---|---|---|---|---|---|
| *Exam.: Example, Comp.: Comparative, Pres.: Pressure | | | | | | | | |

As apparent from the results of Table 1, it can be understood from the results of Examples 1 to 3 and Comparative Example 1, that the reaction under pressure gives higher production rate of heavy hydrogen in the formation reaction for heavy hydrogen gas of the present invention. It is also understood from the results of Examples 1 to 3 and Comparative Example 2 that the presence of a catalyst is indispensable to the formation reaction for heavy hydrogen gas of the present invention. It is further understood from the results of Examples 1 and 2 that a larger amount of a catalyst gives a higher production rate of heavy hydrogen in short reaction time. It is also understood from the results of Examples 1 and 3 that a higher reaction pressure gives a higher production rate of heavy hydrogen.

### Examples 4 to 7. Synthesis of heavy hydrogen gas of the present invention

In a 96-mL autoclave serving as a pressure vessel, 1 mL (55 mmol) of deuterium oxide (D₂O) and 20 mg of each of 5% various catalysts were added, and the reaction system was pressurized with hydrogen (hydrogen pressure: 10 atmosphere, system pressure: 11 atmosphere in conjunction with atmosphere pressure) (H₂: 46.7 mmol) and subjected to reaction under stirring at room temperature for a predetermined time. After termination of the reaction, the amount of produced DHO was measured by ¹H-NMR and the amount of D₂ produced from DHO and the D₂ production rate (DHO/D₂O + DHO) were calculated. The results are shown in Table 2.

**[Table 2]**

| | Reaction Conditions | | | | | Reaction Products | | D₂ Production Rate (mol%) |
|---|---|---|---|---|---|---|---|---|
| | 5% Cat. (20mg) | Pres. (atm) | Time | H₂ (mmol) | D₂O (mmol) | DHO (mmol) | D₂ (mmol) | |
| Ex.4 | Rh/C | 11 | 72h | 46.7 | 55 | 47.3 | 23.7 | 86.1 |
| Ex.5 | Pt/C | 11 | 24h | 46.7 | 55 | 41.1 | 20.6 | 74.8 |
| Ex.6 | Ir/C | 11 | 24h | 46.7 | 55 | 36.2 | 18.1 | 65.9 |
| Ex.7 | Ru/C | 11 | 24h | 46.7 | 55 | 37.6 | 18.8 | 68.4 |

| | | | | | | | | |
|---|---|---|---|---|---|---|---|---|
| *Ex.: Example, Cat.: Catalyst, Pres.: Pressure | | | | | | | | |

As apparent from the results of Table 2, it has been found that the formation reaction for heavy hydrogen gas of the present invention can produce heavy hydrogen gas efficiently even when various metal catalysts are used.

### Examples 8 to 11. Catalytic deuteration method of the present invention (catalytic deuteration reaction of trans-cinnamic acid)

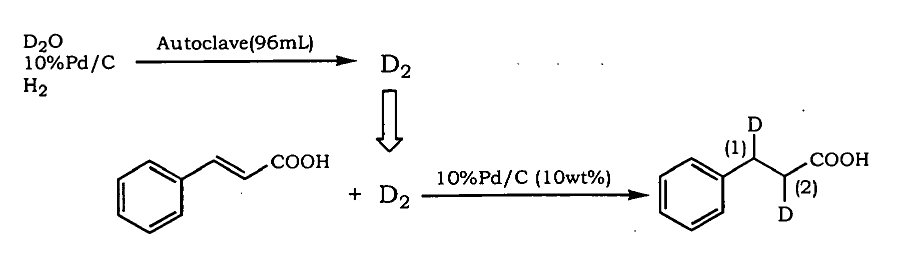

In a 96-mL autoclave serving as a pressure vessel, a predetermined amount of deuterium oxide (D₂O) and a predetermined amount of 10% Pd/C were added, and the reaction system was pressurized with hydrogen to a predetermined pressure (system pressure: pressure obtained by adding atmospheric pressure (1 atmosphere) to the hydrogen pressure) and subjected to reaction under stirring at room temperature for 48 hours. After termination of the reaction, the obtained heavy hydrogen gas (D₂) was collected in a balloon. Then, in a 100-mL eggplant-shape flask, 0.5 mmol of trans-cinnamic acid as a reaction substrate, 3 mL of cyclohexane as a solvent and 10% by weight of 10% Pd/C based on the substrate were added. Thus produced reaction system was replaced with the obtained D₂ gas and subjected to catalytic reduction at room temperature for 6 hours. The obtained results are shown in Table 3.

**[Table 3]**

| | Reaction Conditions for D₂ Formation | | | | Deuteration Ratio (%) | |
|---|---|---|---|---|---|---|
| | D₂O (mL) | Pd/C (mg) | H₂ (mmol) | Pressure (atm) | C1 | C2 |
| Example 8 | 10 | 50 | 23 | 6 | 41 | 40 |
| Example 9 | 10 | 100 | 23 | 6 | 42 | 42 |
| Example 10 | 15 | 30 | 39.8 | 11 | 45 | 42 |
| Example 11 | 15 | 100 | 23 | 6 | 45 | 45 |

| | | | | | | |
|---|---|---|---|---|---|---|
| * Reaction Time for Example 11: 72 hours | | | | | | |

### Examples 12 to 16. Catalytic deuteration method of the present invention (catalytic deuteration reaction for trans-cinnamic acid ethyl ester)

In a 96-mL autoclave serving as a pressure vessel, a predetermined amount of deuterium oxide (D₂O) and 50 mg of 10% Pd/C were added, and the reaction system was pressurized with hydrogen of 5 atmosphere (system pressure: pressure obtained by adding atmospheric pressure (1 atmosphere) to the hydrogen pressure) and subjected to reaction under stirring at room temperature for a predetermined time. After termination of the reaction, the obtained heavy hydrogen gas (D₂) was collected in a balloon.

Then, in a 100-mL eggplant-shape flask, 0.5 mmol of trans-cinnamic acid ethyl ester as a reaction substrate, 3 mL of cyclohexane as a solvent and 10% by weight of 10% Pd/C based on the substrate were added. Thus produced reaction system was replaced with the obtained D₂ gas and subjected to catalytic reduction at room temperature for 6 hours. The obtained results are shown in Table 4.

**[Table 4]**

| | Reaction Conditions for D₂ Formation | | | | | Deuteration Ratio (%) | |
|---|---|---|---|---|---|---|---|
| | D₂O (mL) | Pd/C (mg) | H₂ (mmol) | Pressure (atm) | Time (h) | C1 | C2 |
| Example 12 | 10 | 50 | 23 | 6 | 72 | 46 | 46 |
| Example 13 | 10 | 50 | 23 | 6 | 72 | 47 | 45 |
| Example 14 | 20 | 50 | 20.4 | 6 | 120 | 48 | 47 |
| Example 15 | 10 | 30 | 11.5 | 3 | 48 | 47 | 47 |
| Example 16 | 10 | 50 | 11.5 | 3 | 48 | 49 | 49 |

As apparent from the results of Tables 3 and 4, it has been found that deuterared compounds with a high deuteration ratio can be obtained when various reaction substrates are subjected to catalytic deuteration using D₂ obtained by the method for producing heavy hydrogen gas of the present invention. In addition, as apparent from Example 16, it has been found that heavy hydrogen atoms can be introduced to C1 and C2 almost quantitatively by using the heavy hydrogen gas obtained by the present invention.

It should be noted, as for whether D₂ is produced or not in the method for producing heavy hydrogen gas of the present invention, it can be determined by the deuteration ratio (observed value) of a deuterated reaction substrate obtained by subjecting the reaction substrate to catalytic reduction reaction with the heavy hydrogen gas produced by the method for producing heavy hydrogen gas of the present invention. Namely, provided that the whole reaction system is occupied with D₂ before the catalytic reduction, the theoretical deuteration ratio of C 1 and C2 of the substrate shall be each 50%. On the other hand, provided that the whole reaction system is occupied with DH, the theoretical maximum deuteration ratio of C1 and C2 shall be each 25%. Therefore, considering that each result of Examples 8 to 14 gives a deuteration ratio of the reaction substrate of not less than 40%, it can be understood that the method for producing heavy hydrogen gas of the present invention can produce D₂ efficiently.

Also, as apparent from the results of Example 11, the deuteration ratio of the reaction substrate are each 45%. Namely, provided that the reaction system was occupied with D₂ and H₂ before the catalytic reduction, D₂ and H₂ were present at a rate of 90% and 10% respectively. On the other hand, provided that the reaction system was occupied with D₂ and DH, D₂ and DH were present at a rate of 80% and 20% respectively. It is apparent from this result, therefore, that the method for producing heavy hydrogen gas of the present invention can produce D₂ gas efficiently from deuterium oxide and that even if DH is produced as a byproduct, its abundance ratio is much lower than the production rate of D₂ gas.

### Example 17. Synthesis of a secondary amine by reducible monoalkylation using heavy hydrogen gas

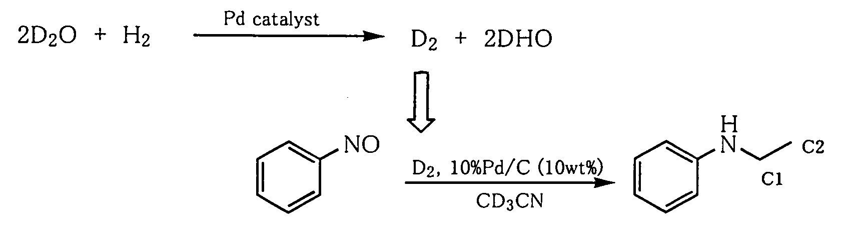

In deuterated acetonitrile (0.75 mL), 61.6 mg (0.5 mmol) of nitrobenzene as a reaction substrate and 6.2 mg of 10% by weight of 10% Pd/C relative to the substrate were suspended, degassed and then subjected to reaction under intense stirring at room temperature for 24 hours while adding the heavy hydrogen gas using the balloon, in which the heavy hydrogen gas obtained in Example 16 is collected. The objective product was obtained by filtering out the catalyst from the obtained reaction solution through a membrane filter (Millipore, Millex (registered trademark) -LH, 0.45 µm) and evaporating off the solvent under reduced pressure. The results are shown in Table 5.

### Example 18. Synthesis of a secondary amine by reducible monoalkylation reaction using heavy hydrogen gas

The objective product was obtained by similar operation as in Example 17 except that 1.5 mmol of acetonitrile as a substrate and 1.0 mL of cyclohexane as a solvent, instead of 0.75 mmol of deuterated acetonitrile, and 20% by weight of 10% Pd/C instead of 10% by weight of 10% Pd/C were used. The results are shown in Table 5.

**[Table 5]**

| | Substrate (mmol) | Solvent (mL) | 10%Pd/C (wt%) | Deuteration Ratio (%) | | Production Rate (%) |
|---|---|---|---|---|---|---|
| | | | | C1 | C2 | |
| Ex. 17 | PhNO₂(0.5) | CD₃CN(0.75) | 10 | 77 | 98 | 89 |
| Ex. 18 | PhNO₂(0.5) CH₃CN(1.5) | Cyclohexane(1.0) | 20 | 64 | 27 | 73 |

As apparent from the results of Table 5, it has been found that a secondary amine compound having a high deuteration ratio can be synthesized by reacting a nitro compound and a nitrile compound using the heavy hydrogen gas obtained by the method for producing heavy hydrogen gas of the present invention.

### Industrial Applicability

Heavy hydrogen gas can be produced in a high production rate to a deuterated solvent according to the method for producing heavy hydrogen gas of the present invention where heavy hydrogen gas is produced by bringing the deuterated solvent into contact with hydrogen gas under pressure in the coexistence with a catalyst selected from among a palladium catalyst, a platinum catalyst, a nickel catalyst, a cobalt catalyst, an iridium catalyst, a rhodium catalyst not coordinated with a ligand and a ruthenium catalyst not coordinated with a ligand. In addition, when a reaction substrate is subjected to catalytic deuteration reaction in the presence of a catalytic reduction catalyst using the heavy hydrogen gas produced by the method for producing heavy hydrogen gas of the present invention, the reaction substrate can be efficiently catalytically deuterated using the heavy hydrogen gas efficiently produced from deuterium oxide which is relatively inexpensive and available, without purchasing expensive heavy hydrogen gas, and the deuterated compound having a high deuteration ratio can be produced.

## Claims

1. A method for producing heavy hydrogen gas, comprising bringing a deuterated solvent into contact with hydrogen gas under pressure in the coexistence with a catalyst selected from a palladium catalyst, a platinum catalyst, a nickel catalyst, a cobalt catalyst, an iridium catalyst, a rhodium catalyst not coordinated with a ligand and a ruthenium catalyst not coordinated with a ligand.

2. The method according to claim 1, wherein the reaction pressure is 2 to 15 atmosphere.

3. The method according to claim 1, wherein the catalyst is not coordinated with the ligand.

4. The method according to claim 1, wherein the catalyst is the palladium catalyst.

5. The method according to claim 4, wherein the palladium catalyst is a palladium carbon.

6. The method according to claim 1, wherein the deuterated solvent is deuterium oxide (D₂O) or tritium oxide (T₂O).

7. The method according to claim 1, wherein the amount of use of the deuterated solvent is 0.1 to 20 molar times relative to the hydrogen gas.

8. A catalytic deuteration method of a compound having a reducible functional group, comprising bringing the heavy hydrogen gas obtained by the method of claim 1 into contact with the compound having a reducible functional group in the coexistence with a catalytic reduction catalyst.

9. The deuteration method according to claim 8, wherein the reducible functional group is a carbon-carbon double bond, a carbon-carbon triple bond, an epoxy group, a halogen atom, a nitrile group, a hydroxyl group bonding to a carbon atom directly bonding to an aromatic ring (hydroxyl group bonding to benzyl position), an carbonyl group, an imino group, an aromatic ring, or a cyclic alkyl group having 3 to 4 carbon atoms.

10. The deuteration method according to claim 8, wherein the catalytic reduction catalyst is the palladium catalyst.

11. The deuteration method according to claim 10, wherein the palladium catalyst is the palladium carbon.
